Europäisches Patentamt

European Patent Office

Office européen des brevets

(11) Publication number: **0 222 914**
**A1**

## (12) EUROPEAN PATENT APPLICATION

published in accordance with Art. 158(3) EPC

(21) Application number: 85905108.8

(51) Int. Cl.⁴: **A 61 K 9/16,** A 61 K 9/00

(22) Date of filing: 11.10.85

Data of the international appli-
cation taken as a basis:

(86) International application number:
**PCT/JP 85/00565**

(87) International publication number:
**WO 86/05393 (25.09.86 86/21)**

(30) Priority: 14.03.85 JP 49224/85

(71) Applicant: **TEYSAN PHARMACEUTICALS CO., LTD., 9,
Nihonbashi-Honcho 2-chome, Chuo-ku, Tokyo 103 (JP)**

(43) Date of publication of application: 27.05.87
**Bulletin 87/22**

(72) Inventor: **KOCHI, Tsuyoshi 14-1, Shinmei 2-chome,
Hino-shi, Tokyo 191 (JP)**
Inventor: **HONDA, Tadao 13-3, Koishikawa 5-chome,
Bunkyo-ku, Tokyo 112 (JP)**

(74) Representative: **Votier, Sidney David et al, CARPMAELS
& RANSFORD 43, Bloomsbury Square, London
WC1A 2RA (GB)**

(84) Designated Contracting States: **BE CH DE FR GB IT LI
NL**

(54) STABILIZED COMPOSITE GRANULAR ANTIBIOTIC PREPARATION.

(57) Composite granular antibiotic preparation composed of orally administrable, rapidly acting granules and slowly acting granules having coated thereon an enteric substance, in which about 1.5 to about 5wt % of water is contained except for water of crystallization bound to the antibiotic molecules.

EP 0 222 914 A1

- 1 -

SPECIFICATION

Technical Field

The present invention relates to a highly stabilized antibiotic compound granular formulation having orally administrative antibiotics as its effective ingredient. More particularly, this invention relates to an antibiotic compound granular formulation which, being a compound granular farmaceutical preparation, contains orally administrative antibiotics having water of crystallization such as amoxicillin (molecular formula, $C_{16}H_{19}N_3O_5S \cdot 3H_2O$; molecular weight, 419.45), and, being a pharmaceutical preparation which is free from coloring while in storage for a long period of time and is stabilized not to be altered its effectiveness, can maintain the effective concentration in the blood serum for a long time when orally administered to humans.

Background of the Art

Orally administrative antibiotics such as amoxicillin have a wide portion of the antibacterial spectrum and display an effective antibactierial activity against gram-positive bacteria as well as gram-negative bacteria and have higherto been widely used in the dosage form of capsule, tablet, or granule as a clinical remedy.

When these antibiotic preparations are administered, they usually have to be administered 4 times a day at intervals of 6 hours because the time for which they keep their effective serum level is comparatively short. It is really a troublesome matter for a patient to take medicine 4 times a day every 6 hours, especially in the case of an outpatient who is not placed under the doctor's direct control it is rather difficult to take medicine regularly and it is reported this is often the case found with a patient forgetting to take medicine after lunch, according to statistical reports made by some hospital people.

Encourged by such a background as mentioned above, various kinds of long-lasting antibiotics preparation intended for maintaining the high serum level have been developed.

As the long-lasting antibiotics, a preparation consisting of a rapidly-soluble component and a sustained-releasing component is known (Japanese Patent Laid-Open Publication Nos. 139713/'77 and 129115/'79).

Furthermore, as the improved type of these long-lasting drug, a compound granular preparation comprising a rapid-acting spherical granule obtained by coating a spherical core material with an antibiotic and a slow-acting spherical granule obtained by coating it with an enteric coating is known (Japanese Patent Laid-Open Publication No. 26816/'83). A compound granular preparation may claim the merit of having a uniform particle size.

For an antibiotic preparation of the sustained-release type, the storage safety may be mentioned to be the most important factor. However excellent a drug may be in its quality, usefulness, and biological versatility, it has no value as a drug if it changes its bioavailability while it is in the course of distribution and in storage at medical institutions.

It is a well known fact that an orally administrative antibiotic such as amoxicillin is a compound which belongs to the penicillins and gradually induces chemical change like hydrolysis and coloring under hot and humid conditions. It is therefore necessary to avoid the presence of any excessive moisture in the final preparations by all means.

In regard to the solid type antibiotics like amoxicillin bulk, it is common that their molecules usually contain water molecules bound them as the water of crystallization, which is rather readily removed from the molecules upon heating or under reduced pressure during the drying stage in the process of producing the pharmaceutical formulation. Generally speaking, so far as the molecular structure is concerned, its water of crystallization itself plays no part in the efficacy of the drug but has a considerable influence on its solubilization behaviour. Since it is often witnessed that the existence of water of crystallization exercises a direct influence on the serum level, that is, the bioavailability by encouraging the absorption upon its administration to a human. Hence, pharmacologically speaking, molecule with crystallization water and anhydrous molecule are two different things.

It is, therefore, an important subject to keep the moisture content in the formulation, especially for the sustained-releasing of antibiotic molecules possessing water of crystallization, within the specific range.

Disclosure of the Invention

The present inventors have prosecuted intensive research on the moisture content of the
pharmaceutical formulation with the purpose of
achieving a constant bioavailability of the antibiotic compound granular formulation comprising
rapid-acting granule and slow-acting granule,
with the result of achieving the present invention.
The present invention is to provide a stabilized antibiotic compound granular formulation
characterized in that the antibiotic compound
granular formulation, which comprises orally
administrative rapid-acting antibiotic granules
and slow-acting granules obtained by coating
said rapid-acting granules with an enteric coating material, is made to have the moisture content of about 1.5 to 5% by weight except for the
water of crystallization bound to the molecules
of the antibiotics.
Drugs that are used in this invention are
orally administrative antibiotics. Examples of
such antibiotics include amoxicillin, cephalexin,
minocycline, cefadroxil, ampicillin, cloxacillin,
dicloxacillin, flucloxacillin, phosphomycin,
cefaclor, cefradine, and a mixture of amoxicillin
and clavulanic acid. The derivatives of these
antibiotics such as their salts and esters may
also be mentioned as the drugs to be used in this
invention. These antibiotics can be used as a
mixture of two or more of then as case may require.

These antibiotics are usually used in the form of a hydrate. In this invention, amoxicillin trihydrate is used desirably.

The rapid-acting orally administrative antibiotic granule in this invention involves granules which are made of drugs only or which are made of drugs and any excipients selected from such vehicles as glucose, lactose, and corn starch, such binders as gum arabi, tragacanth gum, hydroxypropyl cellulose, and polyvinyl pyrrolidone, and such lubricant as magnesium stearate according to any publicly known manufacturing technique. The granules may also be such ones as those made of a core material selected from such carbonhydrates as lactose, fine granulated sugar, and sucrose, and such inorganic substances as calcium phosphate and silicic acid anhydride and a drug which coats the core material. This technique of manufacturing a perfect round granule by coating the spherical core material with a drug is preferable in that it allows the drug to effect the coating uniformly and efficiently. The perfect round core materials can be obtained by covering or coating the crystals or fine grains of such carbonhydrates as lactose, fine granulated sugar, and sucrose or such inorganic substances as calcium phosphate and silicic acid anhydride with any of the aforementioned carbonhydrates, inorganic substances, or such polymers as corn starch and starch while the mass of the fine core materials are being rotationally circulated by means of centrifugal force and jet air stream in the vessel of a centrifugal fluidizing coating and granulating apparatus. Spherical core materials of uniform particle size obtained by sifting out non-standard particle are used desirably.

In obtaining spherical core materials, the amount of the covering or coating material is sufficient when used one to three times the amount of the crystals or fine grains of carbonhydrates or inorganic substances. In the covering or coating process, a binding solution such as an aqueous solution of polyvinyl pyrrolidone or isopropyl alcohol solution of hydroxypropyl cellulose is sprayed all over the crystals or fine grains of carbonhydrates or inorganic substances, which are simultaneously applied with the aforementioned covering or coating material. They are then dried and sifted to obtain spherical core materials of comparatively uniform particle size and because of their perfectly round spherical shape they set forth the merit of achieving the succeeding process of drug coating and enteric coating with uniformity and faultless.

No limit is placed on the method of covering the surface of a core material or spherical core material with the drug and any publicly known method such as spray coating may be adopted; however, the aforementioned centrifugal fluidizing coating method is preferable. Concretely describing, a prescribed quantity of the core materials or spherical core materials are placed in a centrifugal fluidizing granulating apparatus. While letting these core materials or spherical core materials form a fluidizing doubhnut ring motion by the rotation of the rotor together with the air stream blown into through the slit between the stator and the rotor of the granulating apparatus to lift them to an adjusted height, where the core materials or spherical core materials are, where the core materials are moistened with a binding solution prepared by dissolving a gastrically soluble organic polymer such as methyl cellulose, hydroxypropyl cellulose, polyvinyl pyrrolidone, and polyvinyl alcohol in water or low bioling organic solvents sprayed from the spray gun located above them. At the same time, the drug or excipients powder containing the active ingredient is sprayed uniformly over the core materials from the coating powder duct located at the upper part of the granulating apparatus to have the moistened core materials or spherical core materials covered with it and dried. When such procedure is conducted for a certain period of time, the rapid acting granules of desired particle size can be obtained.

With regard to the rapid-acting granules of this invention, the ordinary granules, which are made of the drug only or the drug and vehicles, binders, and lubricants according to a publicly known method, may be covered or coated with the abovementioned gastrically soluble organic polymer.

The quantity of an active ingredient to be contained in the rapid-acting granules of this invention os usually 20 to 80% by weight.

The rapid-acting granules of this invention are thus obtained; however, these rapid-acting granules may be covered with a protective coating of sugar or other organic polymers so far as such coating satisfies the rapid action of the granules by rapidly dissolving in the gastric juice.

The slow-acting granules of this invention can be obtained by coating the rapid-acting granules obtained by the abovementioned method with entric coating materials. As the enteric coating materials, such substances that dissolve at pH value of 5.5 or more are preferable, and those that dissolve at pH value of 6.0 or more are especially preferable for the improved sustained release. Examples of such enteric coating materials include cellulose acetate phthalate, cellulose acetate succinate, methyl cellulose phthalate, ethylhydroxy cellulose phthalate, hydroxypropyl methyl cellulose phthalate, and methyl methacrylate-methacrylic acid copolymers. No limit is specifically placed upon the method of coating the enteric coating material and any of the publicly known methods is applicable. The coating is desirable to be performed in the centrifugal fluidizing coating machine mentioned above. As the solvent, organic solvents such as ethanol, isopropanol, methylene chloride, and acetone are used singly or mixedly besides water. The amount of coating ranges from 5 to 20% by weight, preferably from 8 to 16% by weight, of the rapid-acting granules. In case where spherical granules are used, the present invention may well claim the merit of forming a uniform coating with a less amount of material as mentioned before.

Thus obtained rapid-acting granules (gastric granules) and enteric coated granules (enteric granules) are mixed at a proper ratio to give a compound granular formulation.

The content of the antibiotics in the compound granular formulation is usually in the range of 20 to 75% by weight, preferably 30 to 65% by weight.

If the content of the active ingredient exceeds 70% by weight, the ratio of excipients which are necessary for granulating the ingredients becomes too small to give the granules enough medical function and physical properties. On the contrary, if the content of the active ingredient is 20% by weight or lower, the dose of the drug increases wastefully, both cases being undesirable.

The mixing ratio between the rapid acting granules and the enteric granules is a very important factor to exercise a strong influence on the behavior of the durability of the concentration of antibiotics such as amoxicillin in the blood serum upon the oral administration of the granular formulation. Therefore their mixing ratio, or potency ratio of the respective granules should be determined through careful in vivo tests such as the bioavailability test conducted with healthy subjects.

It is desirable for the whole granules to contain 20 to 80% by weight, preferably 20 to 50% by weight, of gastric granules in order to obtain a sufficient serum level in the early stage after administration.

Thus obtained compound granular formulation is subjected to the drying process to possess its moisture content adjusted to about 1.5 to 5% by weight except for the water of crystallization bound to the molecules of antibiotics.

In the case of the granules of high moisture content stored at 40°C for only 2 months or so, it loses its potency by 10% or more and also starts coloring in tints of light yellow to yellowish green undesirably.

Moreover, in case of being dried excessively, the dissolution of antibiotics into the digestive juice decreases due most probably to the lowered water solubility resulting from the considerable loss of water of crystallization contained in the antibiotics, thus presenting the undesirable wide variation of bioavailability.

It is especially preferable to keep the moisture content ranging from about 3 to about 5% by weight. The drying of a compound granular formulation may be effected according to the method in which the compound granular formulation is dried, for instance, at 60°C under reduced pressure (5mmHg or lower) for several hours or according to another method in which the compound granular formulation is placed in the hot air drier and dried in the hot air stream kept at approximately at 40°C for several hours.

The compound granular formulation bears the residual organic solvent which is used in the course of manufacturing process besides free water content and it is recommendable to substantially remove all such moistening substances in the aforementioned drying process.

It is preferable to apply an antistatic substance to the surface of the compound granular formulation of this invention.

It is axiomatic from the explanation given above that the compound granular formulation of the present invention should be free from an excessive water, which, however, makes the granules in the compound granular formulation, especially the enteric coated granules are ready to be statically electrified in the dry atmosphere since they are covered with a polymer coating which is relatively hydrophobic. A remarkably intensive static electricity is often seen generated under the conditions where the mixed compound granules are vigorously shaken to cause friction. It is therefore desirable to have the compound granular formulation of the present invention, especially its entaric granules, coated with an antistatic material.

As the antistatic materials, inorganic salts such as talc, magnesium stearate, and silicic acid anhydride; nonionic or anionic surface active agents such as polyoxyethylene alkyl ether or its fatty acid amide, N-acyl amino acid or its salt, and polyoxyethylene alkyl ether acetate; and surface active agents such as quaternary ammonium salt of benzalkonium chloride may be mentioned.

As for the methods of coating the granules with an antistatic material, a method of covering over the surface of the whole compound granules with such an antistatic material as talc or magnesium stearate in an amount of 0.3 to 1.0% by weight, a method of having an antistatic material mixed uniformaly in the enteric coating material in an amount of 1 to 10% by weight; and a method of combining these methods are desirably adopted.

In designing the dosage form for the granular formulation of this invention, it is a usual practice to divide the formulation into a single-dose sachet fitted for its use and dosage. The packing process includes two cases, one is a case where the formulation makers carry out the wrapping by themselves before the delivery or shipment of the formulation, and another is a case, called "in hospital packing", where employed pharmacists are engaged in the packing of the formulation with the use of a simple packing apparatus in the hospital for supplying patients with drugs. For a compound granular formulation of the present invention, both cases involve a serious problem if the two types of granules are made to cause the uneven distribution owing to the generated static electricity mentioned above just before they are put into sachets.

This problem can be effectively solved by covering the compound granular formulation, especially its slow-releasing granules, with an antistatic material as explained in the above.

The concept of granule as referred to in this invention includes so-called granules and fine grains whose particle diameter usually ranges from 50 to 500 micrometers.

As described in the above, the compound granular formulation of the present invention is substantially influenced by its coexisting water, though it seems to have no relation with the effectiveness of the formulation. This is the reason its moisture content is restricted specifically in this invention. The compound granular formulation of this invention has the following outstanding merits.

(i)     The compound granular formulation of this invention does not cause coloring and keeps its initial stability even when it is stored for a long period of time.

(ii)     Its active ingredient antibiotic displays the excellent solubility and absorbability upon its administration.

(iii)     It scarcely generates static electricity, and accordingly its packing operation can be carried out very efficiently.

Best Mode of Carrying Out the Invention

The best mode of carrying out the present invention is illustrated by the following examples, which are not intended to be limitative.

Example 1

(i) Preparation of the compound granular formulation:
Fine granulated sugar of appropriate particle size (about 300 to 500 micrometers) weighing 2.5 kg was placed in a centrifugal fluidizing coating and granulating apparatus with a capacity of about 10ℓ. While the rotor was rotated at 150 to 170 rpm, air was blown into the vessel through the slit. The temperature of the air was 50 ∿ 70°C. While the granulated sugar particles were circulated in the vessel, corn starch was added thereto by degrees from the powder duct and a 1% aqueous solution of polyvinyl pyrrolidone was sprayed upon them from the spray gun at a flow rate of 0.05ℓ/min. About 2.5 kg of corn starch was applied to the granulated sugar particles in this process which was continued for 30 minutes. They were dried to give 4 kg of spherical core material of uniform particle size (about 500 to 840 micrometers).

The obtained spherical core material was again placed in the abovementioned centrifugal fluiding coating and granulating apparatus and while being centrifugally circulated in the vessel, the spherical core material was dusted with a mixture of fine powder of amoxicillin and starch and was also sprayed at the same time with a 1% aqueous-isopropyl alcohol solution of hydroxy propyl cellulose to effect the granulation of spherical core material.

The granulating operation was stopped when an appropriate amount (about 50% by weight) or amoxicillin coating was completed and was followed by drying to give a proper dryness, thus producing gastric granules.

Thus obtained gastric granules were sifted and a workable amount (about 6 kg) of the granules measuring 710 to 1,000 micrometers in particle size were placed in the same granulating apparatus to be covered with an enteric coating. This coating operation was carried out by rotating the rotor at 150 to 170 rpm in the air blown at 30°C.

In making an enteric coating, a vinyl copolymer having a carboxylic acid group on its side-chain (copolymer of methacrylic acid and methyl methacrylate) was used and a plasticizer (ester of fatty acid monoclyceride) was contained in the coating. A mixture of isopropyl alcohol and methylene chloride was used as the solvent for the coating.

The coating solution was sprayed at a ratio of 0.03ℓ/min upon the surfaces of rapid-acting granules by degrees to form about 10% by weight of enteric coating uniformly. The solvent which had been used in preparing the coating was mostly evaporated and carried out of the vessel during the course of granulating.

Thus obtained two types of spherical granules (gastric and enteric granules) are mixed at various mixing ratios to make compound granular formulations. In the aforementioned process of production, especially in the stage of granulating gastric granules, the respective quantities of spherical core material, effective ingredient, and fillers may be varied to make the granules contain the desired amount of amoxicillin so that compound granular formulations each having its own specific delayed action may be prepared by varying the mixing ratio between the two types of granules.

(ii) Determination of the properties of the respective granules obtained according to the aforementioned process for producing the compound granular formulations:

(A) Small amounts of the gastric granules and enteric granules sampled immediately after production were respectively dried for 3 hours in a dryer under reduced pressure of 1 to 2 mmHg at 60°C (under this drying condition the whole moisture contained in the granules including the water of crystallization of the antibiotics is to be removed). The gastric granules showed loss on drying of 25.1% by weight and the enteric granules showed 22.0% by weight. It may therefore be said that the whole moisture content of the gastric granules was 25.1% by weight and that of the enteric granules was 22.0% by weight

(B) The amoxicillin content of the two types of granules immediately after production was determined by the cylinder-plate assay method for amoxicillin prescribed in the Japanese Antibiotics Drug Standards.

B. subtilis ATCC 6630 were used as the inoculated bacteria and the cultivating medium was prepared by adding 5.0g of peptone, 3.0g of beef extract, and 15.0g of agar to water to make a total of 1,000ml (pH6.5), which was then sterilized at 121°C for 15 minutes. The specimen solution and the standard solution were respectively adjusted with the use of a 1% phosphate buffer (pH6.0). The concentrations were 10 micrograms (potency) /ml and 2.6 micrograms (potency)/ml respectively.

The compound granular formualtion prepared by mixing the two types of granules fresh from the granulating apparatus to make the potency ratio of 3:7 (gastric granules: enteric granules) was placed in the hot air dryer to prepare the compound granular formulations of three degrees of dryness (Table 1, Nos. 2, 3, and 4) in the stream of hot air heated up to 40°C, or by the vacuum drying conducted at 40°C.

The loss on drying (corresponding to the whole moisture content inclusive of water of crystallization of the antiviotics in the compound granular formulation) of the respective specimen formulations were determined at 60°C under reduced pressure (1 to 2 mmHg) for 3 hours. The total potency of amoxicillin contained in the respective specimen formulations was determined after they were thoroughly pulverized and extracted with water of pH 7.

Thus obtained four groups of compound glanular formulations of different dryness are given in Table 1 to show their respective properties.

Table 1

| Physical properties | No. | | | |
|---|---|---|---|---|
| | 1 | 2 | 3 | 4 |
| Drying process | Not dried | Vacuum dried at 40°C | Hot air dried | Hot air dried |
| Weight before drying (g) | 5.0 | 5.0 | 5.0 | 5.0 |
| Loss on drying A (%) | 23.3 | 10.5 | 12.4 | 14.7 |
| Amoxicillin potency (g) * | 3.1 | 3.7 | 3.6 | 3.5 |
| Amoxicillin content (%) | 62 | 74 | 72 | 70 |
| Moisture content equivalent to water of crystallization, B (%) | 8.5 | 9.5 | 9.3 | 9.0 |
| Moisture content of complex granules except for water of crystallization (%), A - B (%) | 15.3 | 1.0 | 3.1 | 5.7 |

* The value calculated as the molecules containing water of crystallization based on the determined potency.

- 18 -

(C)   The compound granules belonging to the aforementioned four groups were respectively packed in polypropylene-alminum laminated film sachets, each containing 5g, and sealed.

These sachets were kept in a oven maintained at 40°C for 3 months and the granules of the respective groups were examined to know how their physical properties had changed. The results are shown in Table 2 below.

Table 2

| No. | Description | Loss on drying | Time of disintegration * | Amoxicillin potency |
|-----|-------------|----------------|--------------------------|---------------------|
| 1 | Changed to yellowish green | No loss | minutes 5.9 | 2.6 g |
| 2 | No change | Ditto | 5.5 | 3.7 |
| 3 | No change | Ditto | 5.8 | 3.6 |
| 4 | Changed to yellow | Ditto | 5.6 | 3.5 |

*   Determined according to the disintegration test of the Japanese Pharmacopoeia.  Weighing 0.10g of each specimen into tubes of basket, using a buffer of pH 6.8 (the 2nd solution) as the test solution, the test was conducted by moving the baskets up and down while being kept at 37°C on the disintegration tester and the time required for the complete disintegration of the specimen was measured.

As seen from Table 2, the specimen No. 1 which contained much moisture left undried caused a remarkable coloring and also showed a 15% loss of potency. No remarkable difference was seen among the specimens with regard to the time of disintegration. The granules of specimen No. 4 showed an overall change of color to yellowish tints probably due to its high A-B% of 5.7% as compared with Nos. 2 and 3. No. 2 showed a slow dissolution as described later.

Example 2

Based on the process for granulating described hereinabove, a compound granular formulation having about 50% potency of amoxicillin was prepared according to the same method as Example 1. The prepared compound granular formulation was divided into four groups, each group having a different degree of dryness.

The physical properties of the compound granules thus prepared are shown in Table 3.

Table 3

| Physical Properties | No. | | | |
|---|---|---|---|---|
| | 5 | 6 | 7 | 8 |
| Drying process | Not dried | Vaccum dried at 40°C | Hot air dried | Hot air dried |
| Weight before drying (g) | 5.0 | 5.0 | 5.0 | 5.0 |
| Loss on drying A (%) | 20.8 | 5.0 | 10.5 | 13.5 |
| Amoxicillin potency (g) | 2.2 | 2.65 | 2.5 | 2.4 |
| Amoxicillin content (%) | 44 | 53 | 50 | 48 |
| Moisture content equivalent to water of crystallization, B (%) | 5.7 | 6.9 | 6.5 | 6.2 |
| Moisture content of complex granules except for water of crystallization (%), A - B (%) | 15.1 | -1.9 | 4.0 | 7.3 |

The compound granules of these 4 groups were respectively packed in stick type sachets of impermeable aluminum laminated film, each containing 1.2g of compound granules.

The sachets were left as they were at room temperature for 27 months to study how their physical properties would change according to Example 1.

The granules of specimen No. 5, which had not been dried, took on a brownish color and their amoxicillin potency was 2.0g which meant a 10% drop. Yellow coloring was distinctly observed on the granules of specimen No. 8 (whose moisture content, reducing the water of crystallization therefrom, was 7.3%, which meant an insufficient dryness) and their amoxicillin potency dropped by about 4%.

Contrary to these sepecimens, the dryness of the compound granules of specimen Nos. 6 and 7 seemed satisfactorily enough. No change was observed in their description 27 months after their production. They kept their stability satisfactorily showing only 1 to 2% loss of their amoxicillin potency. Specimen No. 6 showed poor rate of dissolution as described later.

Example 3

In conformity with the process of granulation mentioned above, compound granules having amoxicillin potency of 30% or thereabout were produced according to Example 1. The compound granules were divided likewise into 4 groups, each undergoing the differently controled drying. The physical properties of the respective compound granules are shown in Table 4.

Table 4

| Physical properties | No. | | | |
|---|---|---|---|---|
| | 9 | 10 | 11 | 12 |
| Drying process | Not dried | Vacuum dried at 40°C | Vacuum dried at 40°C | Hot air dried |
| Weight before drying (g) | 5.5 | 5.0 | 5.0 | 5.0 |
| Loss on drying A (%) | 18.6 | 4.5 | 9.5 | 11.9 |
| Amoxicillin potency (g) | 1.46 | 1.7 | 1.6 | 1.6 |
| Amoxicillin content (%) | 29 | 34 | 32 | 32 |
| Moisture content equivalent to water of crystallization, B (%) | 3.8 | 4.4 | 4.2 | 4.2 |
| Moisture content of complex granules except for water of crystallization (%), A - B (%) | 14.8 | 0.1 | 5.3 | 7.7 |

As seen in Examples 1 and 2, the changes of physical properties after an elapse of 27 months were examined. Both specimens Nos. 9 and 12 showed significant coloring and heavy drop of potency and it was doubtful whether they would be useful as the drug. While specimen Nos. 10 and 11 showed no coloring and the loss of potency was in the range of only 1 to 2%, indicating that they remained very stable.

Specimen No. 10 showed a poor velocity of dissolution.

Example 4

Specimen Nos. 2 and 3 shown in Table 1 and Nos. 6 and 7 in Table 3 were put to the dissolution test.

The dissolution test was made according to the Japanese Pharmacopoeia, general rules for general tests, 1st method (rotating basket method). Precisely 0.7g of the compound granules was weighed into a dissolution testing apparatus (NTR-5S3 manufactured by Toyama Industries K.K.). As the test solution, 900mℓ of an acid buffer solution of pH 1.2 was first used, and after the gastric granules were completely dissolved in the basket rotating at 100 rpm, the test solution was changed with 900mℓ of a slightly alkaline buffer solution (KH$_2$PO$_4$-NaOH type) of pH 7.5, with use of which the enteric granules were made to dissolve in the basket rotating at 100 rpm to trace the dissolving behavior of amoxicillin as time passed. The determination of amoxicillin was conducted by comparing its absorbancy measured at 272nm with the absorbancy of a standard solution with the known concentration. The ratio of dissolved amount of amoxicillin contained in the enteric granules to its theoretical amount is shown in Table 5.

Table 5  Dissolution ratio (%)

| Specimen | 5 min. | 10 | 15 | 20 | 30 | 40 | 60 |
|----------|--------|----|----|----|----|----|----|
| No. 2 | 40 | 61 | 72 | 85 | 95 | 99 | 102 |
| No. 3 | 63 | 92 | 98 | 100 | 99 | 97 | 101 |
| No. 6 | 35 | 60 | 68 | 82 | 98 | 100 | 99 |
| No. 7 | 60 | 88 | 99 | 101 | 102 | 99 | 100 |

The mean based on n=3

No. 2 and No. 6, which seemed to have a considerable amount of water of crystallization removed from amoxicillin due to their excess drying, showed a poor rate of dissolution in water as compared with No. 3 and No. 7.

Also, with regard to No. 6 and No. 7, the dissolution behavior in the dissolution test conducted by use of the aforementioned test solution of pH 1.2 was observed as the time proceeded. The result is shown in Table 6.

Table 6  Dissolution ratio (%)

| Time / Specimen | 1 min. | 3 | 5 | 7 | 10 | 15 | 20 | 40 | 60 |
|-----------------|--------|---|---|---|----|----|----|----|----|
| No. 6 | 38 | 58 | 93 | 95 | 98 | 100 | 100 | 103 | 105 |
| No. 7 | 54 | 77 | 102 | 100 | 103 | 102 | 105 | 110 | 118 |

The mean based on n=3.

In was also noticed that No. 6 showed a considerably slow rate of dissolution in the observation of the dissolution behavior of its gastric granules as in the case of its enteric granules.

Example 5

Specimen Nos. 6 and 7, each weighing 1.2g (about 500mg of amoxicillian potency), were given to 6 healthy subjects (male) in fasting state to determine the amoxicillin concentration in the blood serum as the time passed according to the cross-over test made in two groups of three subjects.

The drug concentration in the blood serum was determined according to the cylinder-plate assay method in which B. subtilis was used as the inoculum.

The result: No. 7 showed C-max (macimum concentration in the serum) of 4.72μg/mℓ, T-max (time required for reaching C max) of 4.0 hours, and 12-hour AUC (area under the concentration/ time curve) of 25.0μg/mℓ·hr, while excessively dried No. 6 failed to satisfy the desired concentration in the serum and showed C-max of 2.38μg/mℓ, T-max of 6.5 hours, and AUC of 15.6 μg/mℓ·hr, indicating that it had the very unsatisfactory absorptive action.

The foregoing fact clearly shows that excessive drying exercises a strong influence on the bioavailability of the drug and makes the drug having the same potency less efficacious.

Example 6

About 10g each of the compound granules of No. 10 and No. 11 shown in Table 4 was placed in a polyethylene bag separately and shaken for several minutes. Both compound granules generated static electricity and the granules stuck to the inner walls of the bag and it was hardly possible to let all of them fall to the bottom of the bag.

Contrary to the above, the compound granules of No. 9, which had not been dried, had a loss on drying of 18.6% indicating a high moisture content. They scarcely generated static electricity and only several granules were found sticking to the inner walls of the bag in the same treatment as the above.

In the next experiment, the compound granules of No. 10 and No. 11 were covered with about 0.3% by weight of magnesium stearate dust or talc dust. Both were very fine dust and it was easy to make the dust uniformly cover the granules by placing it in the cotton bag together with the granules and shaking. When these two groups of granules were separately placed in a polyethylene bag and vigorously skaken, no generation of static electricity was observed and absolutely no granules were found stuck to the inner walls of the polyethylene bag. Next, about 1% by weight talc dust was sprinkled over the complex granules of No. 10 which had generated static electricity and stuck to the inner walls of the polyethylene bag. When such dust sprinkling was separated several times, the sticking granules were removed from the walls easily and all the granules settled down at the bottom of the bag

In the abovementioned experiment, it was made clear that the compound granules can be mixed uniformly by covering their surface with a slight amount of antistatic material.

Example 7

Talc dust was mixed into the ingredients of enteric coating, which was referred to in the preparation of the compound granular formulation in Example 1, to be in an amount of 3% by weight of the compound granules, thus obtaining compound granules having approximately the same properties as specimen No. 7 listed in Table 3.

The obtained compound granules were subjected to the experiment conducted with the use of a polyethylene bag as described in Example 6 to observe how static electricity would be generated.

The quantity of the granules which stuck to the inner walls of the polyethylene bag decreased to about 1/3 when compared to the compound granules of No. 7 whose enteric coating contained no talc dust.

Example 8

No. 13 compound granules, which were almost similar in physical properties to No. 7 compound granules of Table 3, were formulated. In preparing the compound granules this time, the enteric coatings were made to contain about 3% by weight of talc dust and the whole compound granules had

- 28 -

their surfaces covered with 0.2% by weight of talc dust.

These two types of granules are compared in Table 7.

Table 7

| No. | Moisture content except for water of crystallization (%) | Amoxicillin content mg (potency) * | | Anti-static material |
|-----|------|-----|-----|-----|
| 7 | 4.0 | 500 | In the rapid-acting granules   150 | Not contained |
|   |   |   | In the slow-acting granules   350 |   |
| 13 | 4.0 | 500 | In the rapid-acting granules   150 | Contained |
|   |   |   | In the slow-acting granules   350 |   |

\*   Theoretical value per 1.2g of the complex granules.

Each of the two types of compound granules was packed by use of a stick-packing apparatus KC-135S type manufactured by Komatsu Seisakusho to obtain 1000 sachets of 1.2g, from which 10 sachets were picked up by random sampling respectively and subjected to the dissolution test in two types of aqueous solutions having different pH values according to Example 4.

The respective solutions were determined by cylinder-plate assay with the use of B. subtilis ATCC 6633 according to the amoxicillin potency test (1) prescribed in the Japanese Antibiotics Drug Standards to examine the potency variations of the gastric granules contained in the respective sachets of compound granules. The result of this content uniformity test is shown in Table 8.

Table 8   Potency of gastric granules
per sachet (mg)

| Specimen No. \ Sachet No. | 1 | 2 | 3 | 4 | 5 | 6 | 7 | 8 | 9 | 10 | $\bar{x}$ | R | σ |
|---|---|---|---|---|---|---|---|---|---|---|---|---|---|
| No.7 | 185 | 151 | 148 | 149 | 193 | 128 | 135 | 152 | 140 | 155 | 153.6 | 65 | 20.52 |
| No.13 | 153 | 154 | 146 | 145 | 140 | 158 | 152 | 157 | 148 | 160 | 151.3 | 20 | 3.41 |

Moreover, the total potency of the compound granules per sachet was determined with regard to 10 sachets random-sampled separately in the same was as above. The result is shown in Table 9.

Table 9    Total potency of compound
granules per sachet (mg)

| Sachet No.<br>Speci-men No. | 1 | 2 | 3 | 4 | 5 | 6 | 7 | 8 | 9 | 10 | $\bar{x}$ | R | σ |
|---|---|---|---|---|---|---|---|---|---|---|---|---|---|
| No.7 | 499 | 509 | 502 | 490 | 512 | 503 | 500 | 489 | 495 | 489 | 498.3 | 23 | 8.11 |
| No.13 | 509 | 505 | 509 | 506 | 504 | 502 | 506 | 503 | 509 | 508 | 506.1 | 7 | 2.60 |

Though the gastric granules and the enteric granules were fed in thoroughly mixed satte to the hopper of the stick-packing apparatus according to the theoritcal formulation, No. 13 granules showed far better content-uniformity than No. 7 granules which were made without antistatic agent.

In case where compound granules are not treated with an antistatic material, much difficulty is experienced in mixing the gastric granules and enteric granules at the prescribed mixing ratio.

Industrial Applications

The antibiotic compound granular formulation provided by the present invention contains a specified amount of water and has various outstanding merits as mentioned below.

(i)    The compound granular formulation of this invention does not cause coloring and keeps its initial stability even when it is stored for a long period of time.

(ii)     Its active ingredient antibiotic displays
the excellent solubility and absorbability
upon its administration.

(iii)     It scarcely generates static electricity,
and accordingly its packing operation can be
carried out very efficiently.

The antibiotic compound granular formulation
of this invention has various advantages as mentioned above and is very useful in curing or
preventing a number of types of infectious diseases.

CLAIMS

1.    A stabilized antibiotic compound granular formulation characterized in that the antibiotic compound granular formulation, which comprises orally administrative rapid-acting antibiotic granules and slow-acting granules obtained by coating said rapid-acting granules with an enteric material, is made to have the moisture content of about 1.5 to 5% by weight except for the water of crystallization bound to the molecules of the antibiotics.

2.    A stabilized antibiotic compound granular formulation according to Claim 1, wherein the surfaces of said compound granular formulation are covered with antistatic materials.

3.    A stabilized antibiotic compound granular formulation according to Claim 1, wherein the surface of said slow-acting granules are covered with antistatic materials.

4.    A stabilized antibiotic compound granular formulation according to Claim 2 o  Claim 3, wherein said antistatic material is an inorganic salt.

5.    A stabilized antibiotic compound granular formulation according to Claim 1, wherein said orally administrative antibiotic is amoxicillin trihydrate.

6.    A stabilized antibiotic compound granular formulation according to Claim 1, wherein said moisture content is about 3 to 5% by weight.

7.      A stabilized antibiotic compound granular formulation according to Claim 1, wherein said rapid-acting granules are granules prepared by coating spherical core material with orally administrative antibiotics.

8.      A stabilized antibiotic compound granular formulation according to Claim 1, wherein the granules do not substantially contain the organic solvent used in the manufacturing process.

# INTERNATIONAL SEARCH REPORT

International Application No. PCT/JP85/00565

**I. CLASSIFICATION OF SUBJECT MATTER** (if several classification symbols apply, indicate all) [3]

According to International Patent Classification (IPC) or to both National Classification and IPC

Int.Cl$^4$  A61K9/16, A61K9/00

**II. FIELDS SEARCHED**

| Minimum Documentation Searched [4] | |
|---|---|
| Classification System | Classification Symbols |
| IPC | A61K9/16, A61K9/00, A61K9/48, A61K 31/43, A61K31/545, C07D499/68, C07D501/22 |

| Documentation Searched other than Minimum Documentation to the Extent that such Documents are Included in the Fields Searched [5] | |
|---|---|
| Jitsuyo Shinan Koho | 1926 - 1985 |
| Kokai Jitsuyo Shinan Koho | 1971 - 1985 |

**III. DOCUMENTS CONSIDERED TO BE RELEVANT** [14]

| Category* | Citation of Document, [16] with indication, where appropriate, of the relevant passages [17] | Relevant to Claim No. [18] |
|---|---|---|
| X | JP, A, 60-1128 (Shionogi & Co., Ltd.) 7 January 1985 (07. 01. 85) Column 15, lower part, line 1 to column 23, line 1 (Family: none) | 1, 5, 6, 7 |
| Y | JP, A, 59-82311 (Shionogi & Co., Ltd.) 12 May 1984 (12. 05. 84) Column 6, upper part, line 7 to column 6, lower part, line 2 (Family: none) | 1, 5, 6, 7 |
| Y | JP, A, 58-26816 (Teysan Pharmaceuticals Co., Ltd.) 17 February 1983 (17. 02. 83) Column 1, line 6 to column 2, line 1 & DE, A1, 3229876 & GB, A, 2103486 & FR, A1, 2511245 | 1, 5, 6, 7 |
| Y | JP, A, 54-129115 (Miyauchi Yasuyo) 6 October 1979 (06. 10. 79) Column 5, upper part, line 16 to column 5, lower part line 6 (Family: none) | 1, 5, 6, 7 |
| A | JP, A, 58-116414 (Yamanouchi Pharmaceutical Co., Ltd.) 11 July 1983 (11. 07. 83) | 2, 3, 4 |

* Special categories of cited documents: [16]
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step
"Y" document of particular relevance; the claimed invention cannot be considered to involve an inventive step when the document is combined with one or more other such documents, such combination being obvious to a person skilled in the art
"&" document member of the same patent family

**IV. CERTIFICATION**

| Date of the Actual Completion of the International Search [2] | Date of Mailing of this International Search Report [2] |
|---|---|
| December 20, 1985 (20. 12. 85) | January 6, 1986 (06. 01. 85) |
| International Searching Authority [1] | Signature of Authorized Officer [19] |
| Japanese Patent Office | |

Form PCT/ISA/210 (second sheet) (October 1981)

| | | |
|---|---|---|
| | Column 6, upper part, lines 17 to 19 & PT, A, 76023 & ES, A5, 518487 | |
| A | JP, A, 60-37984 (Showa Denko Kabushiki Kaisha) 27 February 1985 (27. 02. 85) Column 5, lower part, lines 11 to 15 (Family: none) | 2, 3, 4 |
| A | JP, A, 53-29936 (Takeda Chemical Industries, Ltd.) 20 March 1978 (20. 03. 78) Column 2, line 14 to column 3, upper part, line 15 & DE, A1, | 1 |

**V.☐ OBSERVATIONS WHERE CERTAIN CLAIMS WERE FOUND UNSEARCHABLE [10]**

This international search report has not been established in respect of certain claims under Article 17(2) (a) for the following reasons:

1.☐ Claim numbers_____ because they relate to subject matter [12] not required to be searched by this Authority, namely:

2.☐ Claim numbers_____ because they relate to parts of the international application that do not comply with the prescribed requirements to such an extent that no meaningful international search can be carried out [13], specifically:

**VI.☐ OBSERVATIONS WHERE UNITY OF INVENTION IS LACKING [11]**

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

Form PCT/ISA/210 (supplemental sheet (2)) (October 1981)

0222914

International Application No. **PCT/JP85/00565**

**FURTHER INFORMATION CONTINUED FROM THE SECOND SHEET**

```
2738712 & US, A, 4161527 & FR, B1,
2364654 & GB, A, 1591654
```

**V.☐  OBSERVATIONS WHERE CERTAIN CLAIMS WERE FOUND UNSEARCHABLE [10]**

This international search report has not been established in respect of certain claims under Article 17(2) (a) for the following reasons:

1.☐  Claim numbers_____, because they relate to subject matter [12] not required to be searched by this Authority, namely:

2.☐  Claim numbers_____, because they relate to parts of the international application that do not comply with the prescribed require-
ments to such an extent that no meaningful international search can be carried out [13], specifically:

**VI.☐  OBSERVATIONS WHERE UNITY OF INVENTION IS LACKING [11]**

This International Searching Authority found multiple inventions in this international application as follows:

1.☐  As all required additional search fees were timely paid by the applicant, this international search report covers all searchable claims of the
international application.

2.☐  As only some of the required additional search fees were timely paid by the applicant, this international search report covers only those
claims of the international application for which fees were paid, specifically claims:

3.☐  No required additional search fees were timely paid by the applicant. Consequently, this international search report is restricted to the
invention first mentioned in the claims; it is covered by claim numbers:

4.☐  As all searchable claims could be searched without effort justifying an additional fee, the International Searching Authority did not invite
payment of any additional fee.

**Remark on Protest**

☐  The additional search fees were accompanied by applicant's protest.

☐  No protest accompanied the payment of additional search fees.

Form PCT/ISA/210 (supplemental sheet (2)) (October 1981)